# EUROPÄISCHE PATENTSCHRIFT

(11) **EP 0 623 324 B1**
(45) Veröffentlichungstag und Bekanntmachung des Hinweises auf die Patenterteilung: **17.06.1998**
(21) Anmeldenummer: 94105056.9
(22) Anmeldetag: 30.03.1994
(51) Int. Cl.: A61F 2/46

(54) **Schlagvorrichtung zum Entfernen oder Eintreiben von Schaftendoprothesen mit glattem konischen oder zylindrischen proximalen Ende**
Impacting device for the insertion or the extraction of the stem of a bone endoprosthesis with smooth conical or cylindrical proximal end
Dispositif à frapper pour l'insertion ou l'extraction de la tige d'une ostéoendoprothèse à partie proximale lisse en forme de cône ou de cylindre

(30) Priorität: 02.04.1993 DE 4310834
(43) Veröffentlichungstag der Anmeldung: 09.11.1994
(73) Patentinhaber: Zach, Wolfgang, D-96486 Lautertal (DE)
(72) Erfinder: Zach, Wolfgang, D-96486 Lautertal (DE)

(56) Entgegenhaltungen:
- EP-A- 0 408 109
- WO-A-90/13271
- WO-A-91/06262
- DE-A- 3 505 567

## Beschreibung

Die Erfindung betrifft eine Ausschlagvorrichtung zum Entfernen und Eintreiben von Schaftendoprothesen mit glattem konischen oder zylindrischen proximalen Ende, bestehend aus einer konischen Klemmvorrichtung, mit der eine reibschlüssige Verbindung zum proximalen Ende der Schaftendoprothese herstellbar ist.
Aus WO-A-9106262 ist eine Vorrichtung gemäß dem Obergriff des Anspruchs 1 zum Ein-und Ausschlagen von Schaftendoprothesen bekannt, bei der eine reibschlüssige Verbindung mit dem glatten proximalen Ende der Schaftendoprothese mittels einer konischen Klemmvorrichtung hergestellt wird. Die Klemmvorrichtung besteht aus einem zylindrischen Spannkopf mit einer innenliegenden axial verschiebbaren Spannhülse, die auf dem proximalen Ende der Endoprothese sitzt. Die Spannhülse weist auf ihrer Außenmantelfläche einen Konus auf, über den sie mit Hilfe des Spannkopfes radiai zusammenpreßbar ist.
Die Aufgabe der Erfindung besteht darin, die Ausschlagvorrichtung der im Oberbegriff des Anspruches 1 angegebenen Art insoweit zu verbessern, daß eine sichere, spielfreie und lagestabile reibschlüssige Verbindung zwischen ihr und dem glatten proximalen Ende der Prothese erhalten wird, ohne hierbei in Hinterschneidungen, Schultern, Gewinden, Bohrungen usw. einzugreifen.

Zur Lösung dieser Aufgabe sind bei der Erfindung die im kennzeichnenden Teil des Anspruches 1 angegebenen Merkmale vorgesehen, wobei noch in den dem Anspruch 1 folgenden Ansprüchen für die Aufgabenlösung vorteilhafte und förderliche Weiterbildungen beansprucht sind.
Die Ausschlagvorrichtung weist also eine Klemmvorrichtung auf, die aus einem zylindrischen Spannkopf mit eingelegtem Klemmring besteht. Dieser Klemmring liegt mit seiner Innenfläche am proximaien Ende der Endoprothese an und weist auf seinem Außendurchmesser einen Doppelkonus auf, über den der Klemmring mit Hilfe des Spannkopfes radial zusammenpreßbar ist. Zur Schaffung einer reibschlüssigen Verbindung wird der Klemmring über den Doppelkonus axial von beiden Seiten verspannt, dadurch radial zusammengepreßt und somit eine gleichmäßige homogene Druckbeaufschlagung der Mantelfläche des proximalen Endes der Prothese erzielt. Der Klemmring ist während des Spannvorganges axial unverschieblich gelagert. Die durch den Reibschluß erreichbare Axialkraft von ca. 16 kN gewährleistet ein sicheres Ausschlagen und Eintreiben der Prothese. Hierzu wird der zylindrische Spannkopf mit dem darin gelagerten Klemmring auf das proximale Ende der Prothese gesteckt und mittels vier stirnseitig angeordneter Zylinderkopfschrauben verspannt.
Um sowohl eine definierte Vorspannkraft zu gewährleisten, als auch ein Nachlassen der Vorspannkraft durch Setzen der Bauteile auf ein Minimum zu reduzieren, sind die Schrauben mit Spannscheiben unterlegt. Die Schrauben werden mit einem handelsüblichen Sechskantstiftschlüssel angezogen, der Spannweg entspricht hierbei dem Federweg der Spannscheiben und beträgt ca. 0,5 mm.
Zum Ausschlagen der Prothese wird ein zylindrischer Stab mit axial verschiebbarem Ausschlaggewicht und Griff mit Anschlagbund in den zylindrischen Spannkopf der Vorrichtung eingeschraubt. Die Stabachse verläuft parallel zur Achse des Prothesenschaftes und weist durch die kompakte Bauweise des Spannkopfes nur einen geringen koaxialen Versatz auf. Stoßkräfte des geführten Ausschlaggewichtes verlaufen dadurch ebenfalls parallel zur Achse des Prothesenschaftes und ermöglichen eine nahezu querkraftfreie knochen- und gewebeschonende Entfernung der Prothese.
Beim Eintreiben der Prothese wird an Stelle des zylindrischen Stabes ein relativ kurzer zylindrischer Bolzen in den Spannkopf eingesetzt und am freien Ende mit dosierten Hammerschlägen beaufschlagt. Der feste spielfreie Sitz der Vorrichtung gewährleistet auch hier eine sichere und feinfühlige Führung der Prothese.
Der eingelegte Klemmring mit Doppelkonus ermöglicht eine sehr kompakte Bauweise des zylindrischen Spannkopfes sowohl im Durchmesser als auch vor allem in der Baulänge. Die Vorrichtung kann deshalb im relativ engen Wundbereich gut gehandhabt werden.
Die Prothese wird durch die Klemmvorrichtung sicher und spielfrei am proximalen Ende gehalten, ohne hierbei in Hinterschneidungen, Schultern, Gewinden, Bohrungen usw. einzugreifen.
Die erfindungsgemäße Vorrichtung ist auf Grund ihrer Teilegeometrie sowie der geringen Zahl ihrer Bauteile einfach und kostengünstig herstellbar.
Zur Anpassung der Vorrichtung an unterschiedlich gestaltete proximale Enden der Prothese, wie z.B. Konen und Zylinder verschiedenen Durchmessers, genügt das Wechseln des lose im Spannkopf eingelegten Klemmringes.
Auf Grund der geringen Herstellungskosten, es muß lediglich der aus drei einfachen Teilen bestehende Spannkopf gefertigt werden, lohnt sich auch die Anschaffung mehrerer Spannköpfe mit unterschiedlichen Klemmringen.

Ein Klemmringwechsel durch den Chirurgen entfällt hierbei.

Die Erfindung wird im folgenden unter Bezugnahme auf die Zeichnung erläutert, die ein vorteilhaftes Ausführungsbeispiel darstellt. Darin zeigen:
**Fig. 1** eine Gesamtansicht der Ausschlagvorrichtung mit Prothese im zusammengebauten Zustand, verkleinert im Maßstab 1:2,
**Fig. 2** eine Ansicht auf die Stirnseite des Spannkopfes, verkleinert im Maßstab 1:2 und
**Fig. 3** eine Schnittdarstellung des Spannkopfes mit Prothesenkonus gemäß Linie A-A in **Fig. 2** im Maßstab 1:1

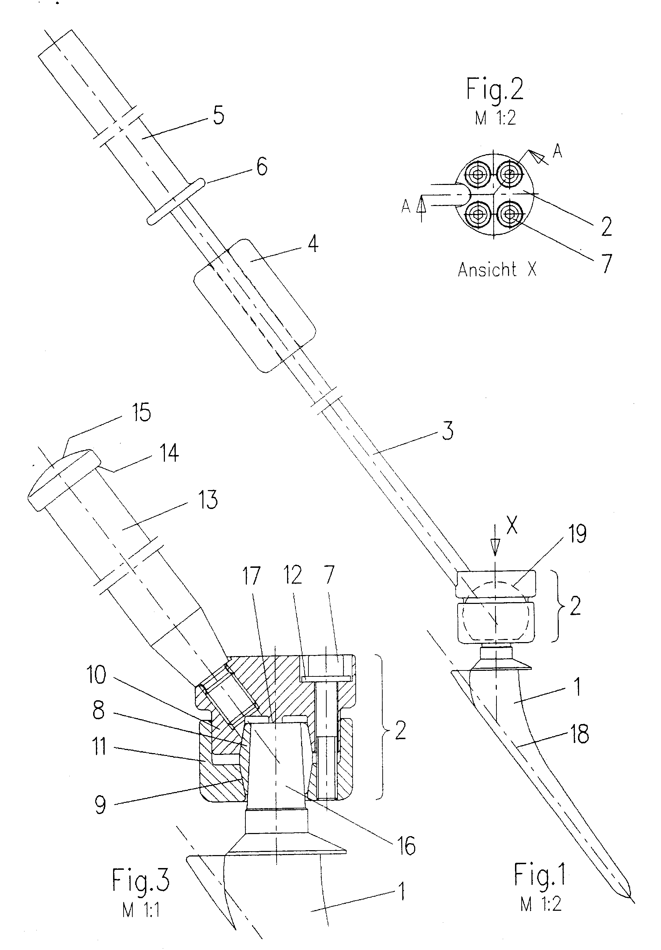

**Fig. 1** zeigt die Schaftprothese **1** mit aufgesetztem Spannkopf **2,** den zylindrischen Stab **3** mit verschiebbarem Ausschlaggewicht **4,** sowie den Griff **5** mit Anschlagbund **6. Fig. 2** stellt die Anordnung der vier Zylinderkopfschrauben **7** in der Stirnseite des zylindrischen Spannkopfes **2** dar. **Fig. 3** zeigt den aufgesetzten Spannkopf **2,** bestehend aus dem lose eingelegten Klemmring **8** mit Doppelkonus **9,** dem Druckflansch **10,** dem Spannring **11,** den Zylinderkopfschrauben **7** mit unterlegten federnden Spannscheiben **12.** Weiterhin in **Fig. 3** dargestellt sind der zylindrische Bolzen **13** mit Schulter **14** und Aufschlagfläche **15** zur Aufnahme dosierter Hammerschläge beim Eintreiben, sowie der Prothesenkegel **16.**

Die axiale Positionierung des Spannkopfes **2** beim Aufsetzen erfolgt durch Anlegen des Anschlagzapfens **17** an die Stirnfläche des Prothesenkegels **16.** Beim Verspannen des Klemmringes **8** über den Doppelkonus **9** mittels Druckflansch **10** und Spannring **11** ist der Klemmring **8** axial unverschieblich gelagert. Der Vorspannweg der Zylinderkopfschrauben **7** entspricht deshalb dem Federweg der Spannscheiben **12** und beträgt lediglich ca. 0.5mm. Die Spannscheiben **12** gewährleisten eine definierte Vorspannkraft der Zylinderkopfschrauben **7,** reduzieren ein Nachlassen der Vorspannkraft durch Setzen der Bauteile auf ein Minimum und verhindern somit ein vorzeitiges Abgleiten des Spannkopfes **2** vom Prothesenkegel **16.**
Je nach Anforderung kann der Klemmring **8** aus Metall oder Kunststoff bestehen, die Innenbohrung kann konisch oder zylindrisch sein, er kann als geschlossener Ring oder längsgeschlitzt offen ausgeführt werden.
Da eine axiale Verschiebung des Klemmringes **8** beim Spannvorgang nicht stattfindet, ist eine Beschädigung der Oberfläche des Prothesenkegels **16** durch Reibverschleiß ausgeschlossen.
**Fig. 1** zeigt sowohl die achsparallele Anordnung des zylindrischen Stabes **3** zur Prothesenschaftachse **18** als auch den geringen koaxialen Versatz beider Achsen.
Die durch das Ausschlaggewicht **4** auf den Anschlagbund **6** einwirkenden Stoßkräfte verlaufen somit ebenfalls parallel zur Prothesenschaftachse **18.**

In Fig. **3** ist an Stelle des zylindrischen Stabes **3** der zylindrische Bolzen **13** mit Schulter **14** und Aufschlagfläche **15** dargestellt. Auch hier verlaufen die durch Hammerschläge auf die Aufschlagfläche **15** beim Eintreiben der Prothese **1** wirkenden Stoßkräfte parallel zur Prothesenschaftachse **18.**

Durch die gleichmäßige homogene Druckbeaufschlagung des Prothesenkegels **16** und der dadurch verringerten Flächenpressung kann die Prothese **1** sowohl aus Stahl als auch aus Kunststoff oder Faserverbundwerkstoffen hergestellt sein.

Die vorteilhafte kompakte Bauweise des Spannkopfes **2** wird durch den Größenvergleich mit der angedeuteten Prothesengelenkkugel **19** in **Fig. 1** verdeutlicht.
Der einfache Aufbau der Vorrichtung, die leichte Zerlegbarkeit und die unkomplizierte Teilegeometrie gestatten eine problemlose gründliche Reinigung und Desinfektion aller Einzelteile.

## Patentansprüche

1. Ausschlagvorrichtung zum Entfernen und Eintreiben von Schaftendoprothesen **(1)** mit glattem konischen oder zylindrischen proximalen Ende **(16),** bestehend aus einer konischen Klemmvorrichtung, mit der eine reibschlüssige Verbindung zum proximalen Ende **(16)** der Endoprothese **(1)** herstellbar ist, wobei die Klemmvorrichtung aus einem zylindrischen Spannkopf **(2)** mit eingelegtem Klemmring **(8)** besteht, der mit seiner Innenfläche am proximalen Ende **(16)** der Endoprothese **(1)** in Anlage bringbar ist und wobei der Klemmring **(8)** mit Hilfe des Spannkopfes **(2)** radial zusammenpreßbar ist, ***dadurch gekennzeichnet,*** daß der Klemmring **(8)** auf seiner Außenmantelfläche einen Doppelkonus **(9)** aufweist, und daß der Spannkopf **(2)** einen mit einem Innenkonus versehenen Druckflansch **(10)** und einen mit einem Innenkonus versehenen Spannring **(11)** aufweist, wobei die Innenkonen von Druckflansch **(10)** und Spannring **(11)** mit dem Doppelkonus **(9)** des Klemmringes **(8)** zusammenwirken, wodurch der Klemmring **(8)** von zwei Seiten verspannbar und radial zusammenpreßbar ist.

2. Ausschlagvorrichtung nach Anspruch 1, ***dadurch gekennzeichnet,*** daß der Klemmring **(8)** mit dem Doppelkonus **(9)** in den entsprechenden Innenkonen des Druckflansches **(10)** und des Spannringes **(11)** spielfrei gelagert ist.

3. Ausschlagvorrichtung nach Anspruch 1 und 2, ***dadurch gekennzeichnet,*** daß der Klemmring **(8)** während des Spannvorganges axial unverschieblich gelagert ist.

4. Ausschlagvorrichtung nach einem der Ansprüche 1 bis 3, **dadurch gekennzeichnet,** daß der Klemmring **(8)** aus Stahl, einem Nichteisenmetall oder Kunststoff bestehen und axial geschlitzt sein kann.

5. Ausschlagvorrichtung nach einem der Ansprüche 1 bis 4, ***dadurch gekennzeichnet,*** daß zur Bestimmung und Sicherstellung der erforderlichen Vorspannkraft am Spannkopf **(2)** Zylinderkopfschrauben **(7)** mit unterlegten Spannscheiben **(12)** angeordnet sind.

6. Ausschlagvorrichtung nach einem der Ansprüche 1 bis 5, ***dadurch gekennzeichnet,*** daß dem Spannkopf **(2)** ein zylindrischer Stab **(3)** zur Aufnahme der mittels Ausschlaggewicht **(4)** erzeugten Schlagimpulse zugeordnet ist

7. Ausschlagvorrichtung nach Anspruch 6, ***dadurch gekennzeichnet,*** daß dem Spannkopf **(2)** an Stelle des zylindrischen Stabes **(3)** ein zylindrischer Bolzen **(13)** mit Aufschlagfläche **(15)** zugeordnet ist, mit dem das Ausschlaginstrument auch als Einschlaginstrument verwendbar ist.

## Claims

1. Driving-out device to remove and to drive in shaft endoprostheses **(1)** having a smooth conical or cylindrical proximal end **(16),** consisting of a conical clamping device, allowing to establish a friction-locking connection to the proximal end **(16)** of the endoprosthesis **(1),** where the clamping device is consisting of a cylindrical chucking head **(2)** with an inlaid clamping ring **(8),** which with its inner surface can be brought into position of the proximal end **(16)** of the endo-prosthesis **(1)** and where, by means of the chucking head **(2),** the clamping ring **(8)** can be compressed radially, **characterized thereby** that on the surface of its outer casing, the clamping ring **(8)** is disposing of a double cone **(9)** and that the chucking head **(2)** is disposing of a pressure flange **(10)** provided with an inner cone and of a chucking ring **(11),** also provided with an inner cone, whereby the inner cones of the pressure flange **(10)** and of the chucking ring **(11)** have a common effect together with the double cone **(9)** of the clamping ring **(8),** whereby the clamping ring **(8)** can be chucked and be radially compressed from two sides.

2. Driving-out device as per claim 1, **characterized thereby** that the clamping ring **(8)** is seated backlash-free together with the double cone **(9)** in the corresponding inner cones of the pressure flange **(10)** and of the chucking ring **(11).**

3. Driving-out device as per claims 1 and 2, **characterized thereby** that during the chucking process, the clamping ring **(8)** is seated unremoveably in axial directions.

4. Driving-out device as per one of the claims 1 to 3, **characterized thereby** that the clamping ring **(8)** can consist of steel, or of a nonferrous metal or of a plastic material and that it can be slotted axially.

5. Driving-out device as per one of the claims 1 to 4, **characterized thereby,** that for determination and securing of the necessary prechucking force, cylinder-head screws **(7)** with underlaid strain washers **(12)** are arranged at the chucking head **(2).**

6. Driving-out device as per one of the claims 1 to 5, **characterized thereby,** that a cylindrical rod **(3)** is coordinated with the chucking head **(2)** serving to absorb the impacting pulses created by the driving-out weight **(4).**

7. Driving-out device as per the claim 6, **characterized thereby,** that in lieu of the cylindrical rod **(3),** a cylindrical bolt **(13)** with an absorption surface **(15)** is coordinated to the chucking head **(2),** with which the driving-out appliance can also be used as a driving-in appliance.

## Revendications

1. Dispositif d'expulsion pour enlever et pour enfoncer des endoprothèses à fût **(1),** ayant une lisse extrémité proximale **(16),** conique ou cylindrique, comportant d'un dispositif de pinçage conique, permettant de créer une jonction sans frottement à l'extrémité proximale **(16)** de l'endo-prothèse **(1),** où le dispositif de pinçage comporte d'une tête de serrage cylindrique **(2)** avec une bague de pinçage logée dedans **(8),** qui avec sa surface intérieure peut être amené en position à l'extrémité proximale **(16)** de l'endoprothèse **(1)** et en permettant de comprimer la bague de pinçage **(8)** radialement à l'aide de la tête de serrage **(2), caractérisé de façon,** que sur la surface de sa chemise extérieure, la bague de pinçage **(8)** dispose d'un double cône **(9),** et que la tête de serrage **(2)** dispose d'une bride à pression avec un cône intérieur **(10)** ainsi qu'une bague de serrage avec un cône intérieur **(11),** où les cônes intérieurs de la bride à pression **(10)** et de la bague de serrage **(11)** donnent un effet commun avec le double cône **(9)** de la bague de pinçage **(8),** en permettant que la bague de pinçage **(8)** peut être serré et comprimé radialement de deux côtés.

2. Dispositif d'expulsion suivant revendication 1, **caractérisé de façon,** que la bague de pinçage **(8)** avec le double cône **(9)** est logée sans jeu dans les cônes intérieurs correspondants de la bride à pression **(10)** et de la bague de serrage **(11).**

3. Dispositif d'expulsion suivant les revendications 1 et 2, **caractérisé de façon,** que pendant le procédé de serrage, la bague de pinçage **(8)** est logé indéplaçablement en sens axial.

4. Dispositif d'expulsion suivant une des revendications 1 à 3, **caractérisé de façon,** que la bague de pinçage **(8)** peut être fabriquée d'acier, d'un métal non-ferreux ou d'une matière plastique et peut être équipée d'une fente axiale.

5. Dispositif d'expulsion suivant une des revendications 1 à 4, **caractérisé de façon,** que pour la détermination et la mise en sécurité de la force de pré-serrage nécessaire, des visses à tête cylindrique **(7)** avec des disques de serrage placés en dessous **(12)** sont arrangées à la tête de serrage **(2).**

6. Dispositif d'expulsion suivant une des revendications 1 à 5, **caractérisé de façon,** qu'à la tête de serrage **(2)** une barre cylindrique **(3)** est coordonnée, servant à absorber les impulsions d'impact produites par le poids d'expulsion **(4).**

7. Dispositif d'expulsion suivant revendication 6, **caractérisé de façon,** qu'à la tête de serrage **(2)** au lieu de la barre cylindrique **(3),** un boulon cylindrique **(13)** est coordonné, disposant d'une surface d'appui **(15),** avec lequel l'appareil d'expulsion est également utilisable comme appareil d'enfoncement.
